# EUROPEAN PATENT APPLICATION

(11) **EP 4 552 658 A1**
(43) Date of publication of application: **14.05.2025**
(21) Application number: 24211502.0
(22) Date of filing: 07.11.2024
(51) Int. Cl.: A61L 2/18, A47L 15/00, A47L 19/00, A61L 2/24, B08B 3/02, B08B 15/02, F28D 1/02

(54) **WASHING AND SANITIZING MACHINE**

(30) Priority: 08.11.2023 SM P202300387
(71) Applicant: Steelco S.p.A., 31039 Riese Pio X (IT)
(72) Inventor: Guarda, Giancarlo, 36028 Rossano Veneto (VI) (IT); Monico, Elena, 31039 Riese Pio X (TV) (IT)
(74) Representative: Petraz, Gilberto Luigi

(57) **Abstract**

Washing and sanitizing machine (10) comprising a containment body (16) that internally delimits a treatment chamber (11) of objects (12), as well as a first circuit (17) for supplying washing and/or sanitizing fluids and a second circuit (18) for discharging said washing and/or sanitizing fluids.

## Description

### FIELD OF THE INVENTION

The present invention concerns a washing and sanitizing machine, in particular for hospital and/or laboratory and/or elsewhere devices and tools where dirty or contaminated devices and tools are produced.

### BACKGROUND OF THE INVENTION

Washing and sanitizing machines are known for devices, tools and/or components, referred to as "objects" hereinafter for the sake of brevity, coming from hospitals, laboratories or elsewhere where dirty or contaminated objects are produced. Said machines include a step of cleaning and washing the objects, followed by steps of sanitizing and drying the objects and the entire chamber that contains them. For example, sanitizing may comprise a thermal disinfection treatment by means of wet vapour, by saturating the washing chamber and maintaining in the saturated chamber an appropriate temperature for a predetermined or variable time.

At the end of the thermodisinfection treatment, for drying the objects, the vapour is removed and hot and dry air is introduced.

A drawback of the state of the art is the energy consumption, and therefore the eco-sustainability, due to the need to filter the air, heat it and circulate it in the washing chamber.

There is therefore the need to perfect a washing and sanitizing machine which can overcome at least one of the disadvantages of the state of the art.

To do this, it is necessary to solve the technical problem of efficiently removing the vapour present in a washing chamber and then drying the objects and the chamber itself, limiting the expenditure of energy.

One purpose of the present invention is to realize a washing and sanitizing machine that reduces the drying energy costs.

The Applicant has devised, tested and embodied the present invention to overcome the shortcomings of the state of the art and to obtain these and other purposes and advantages.

### SUMMARY OF THE INVENTION

The present invention is set forth and characterized in the independent claims.

The dependent claims describe other characteristics of the present invention or variants to the main inventive idea.

In accordance with the above purposes, embodiments described herein relate to a machine for washing and sanitizing objects. According to one embodiment, said machine comprises a containment body which internally delimits a treatment chamber for treating the objects, a first circuit for feeding washing and/or sanitizing fluids and a second circuit for discharging washing liquids. The aforementioned machine comprises a suction fan associated with the aforementioned containment body and configured to extract, by suction, the sanitizing fluids from the treatment chamber. The machine also comprises a further ventilation circuit, configured to compensate for the extraction of sanitizing fluids from the treatment chamber with clean air. Said ventilation circuit is provided with a control valve configured to control the incoming air flow.

Further embodiments concern a method of washing and sanitizing objects in a machine comprising a containment body which internally delimits a treatment chamber for treating the objects themselves. According to embodiments, this method comprises sanitizing the objects contained in said chamber. Said method comprises drying said objects by extracting the sanitizing fluids from the treatment chamber by suction through a suction fan. The method also comprises a ventilation step at the same time or after the drying step, in which, wherein clean air enters the treatment chamber through a ventilation circuit, configured to compensate for the extraction of sanitizing vapours from said treatment chamber with said clean air and provided with a control valve configured to control the incoming air flow.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a schematic representation of a washing and sanitizing machine according to a first embodiment described herein;
- fig. 2 is a schematic representation of a washing and sanitizing machine according to a second embodiment described herein.

We must clarify that in the present description the phraseology and terminology used, as well as the figures in the attached drawings also as described, have the sole function of better illustrating and explaining the present invention, their function being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS OF THE PRESENT INVENTION

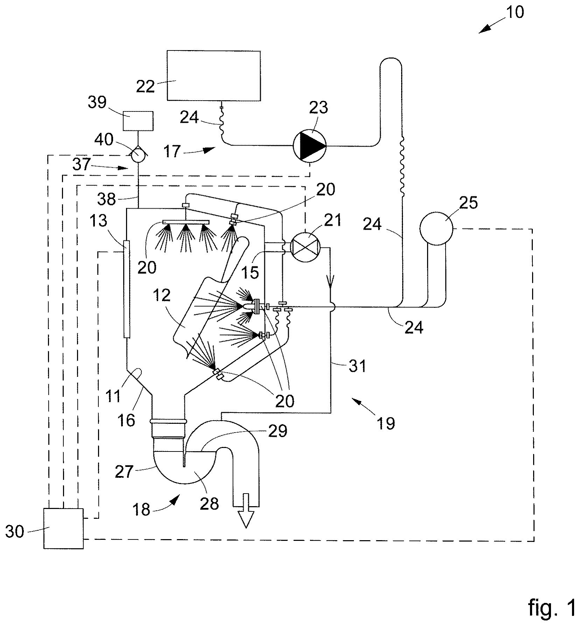

Figs. 1-2 are used to describe embodiments of a machine 10 for washing and sanitizing dirty or contaminated objects, such as for example medical, hospital, laboratory devices, instruments or tools or of other similar or assimilable provenance.

Herein and in the present description the term washing can be understood as washing and/or activity by means of liquids, hot and/or cold with or without the use of chemical agents, the washing cycle being per se known and variable according to the type of objects to be washed.

The term washing liquid will be used with reference to the liquid that is used in the washing cycles, which can be water, or possibly water supplemented with chemical agents. In embodiments, different types of washing liquids can also be used, for example, use can be made of supplemented water for washing and non-supplemented water for any rinsing operations.

The term sanitization can be understood as a thermal disinfection and/or sterilization treatment, typically through the use of hot air and/or vapour or in general cleaning aeriform fluids, for a pre-established or variable time depending on the needs and specific cases. Preferably, the sanitization takes place as a thermal disinfection with hot water vapour. For simplicity of explanation, embodiments will be described below in which the fluid used for sanitization is a vapour, hereinafter also referred to as sanitizing vapour, but this must not be considered a limiting factor.

The machine 10 comprises a containment body 16 which delimits in its inside a treatment chamber 11, in this case for washing and/or sanitization in particular, of objects 12 to be washed because they are dirty or contaminated, for example by chemical agents and/or agents or biological material. The treatment chamber 11 preferably has suitable dimensions and shapes to accommodate inside it one or more objects 12 to be washed, coming from hospitals or laboratories or from elsewhere where devices or objects in general containing, or on which there are, contaminants are produced.

The containment body 16 includes an access door 13 which can be opened/closed to insert and remove objects in/from the treatment chamber 11. In embodiments, the machine 10 comprises a first circuit 17 for feeding washing and/or sanitizing fluids, generally washing liquids and/or sanitizing vapours, and a second circuit 18 for discharging washing liquids.

The containment body 16 is connected to said first 17 and second 18 circuit so that the respective fluids can be suitably dispensed into or removed from the treatment chamber 11. The machine 10 is provided with first injection means 20 mounted on the walls of the containment body 16, inside the treatment chamber 11. The first injection means 20 are means adapted to inject, into the treatment chamber 11, washing liquids and/or sanitizing vapours, for example, but not only, washing nozzles. Said first injection means 20 are favourably connected to the first circuit 17 which, therefore, supplies the necessary liquid to said first injection means 20.

The second discharge circuit 18 may comprise in a known manner a siphon 27 in which a liquid seal 28 is present, the level of which forms a head 29.

The machine 10 comprises a suction fan 21 associated with said containment body 16, for example installed on an external wall of the latter, and in fluidic communication with the treatment chamber 11. Said suction fan 21 is configured to extract, by suction, the sanitizing fluids, for example vapours, from the treatment chamber 11. This suction fan 21 can be advantageously activated at the end of the sanitizing step, to remove the sanitizing vapours present in the treatment chamber 11 and therefore favour the drying of the objects 12.

The machine 10 may include a control unit 30, adapted to control the operation of the machine 10 and consequently also the selective activation of the suction fan 21 as a function of the specific steps of the treatment cycle to which the objects are subjected.

The suction fan 21 can be connected to the treatment chamber 11 by means of at least one opening 15 configured to allow the suction of fluids from the treatment chamber 11.

The suction fan 21 can be mounted on a wall of the containment body 16 different from those where the door 13 is present, for example opposed to it. Alternatively, the suction fan 21 can be mounted on the same wall as the containment body 16 of the door 13.

The suction fan 21 is connected downstream with the second circuit 18 through a third suction circuit 19 so as to discharge, through the at least one opening 15, the sanitizing vapours extracted from the treatment chamber 11. A discharge duct 31 may connect the suction fan 21 downstream of the siphon 27.

The sanitizing vapours present in the treatment chamber 11 can therefore be extracted from the latter by sucking them by means of the suction fan 21 through the at least one opening 15.

The presence of the fan 21 allows said vapours to be extracted from said chamber 11 without the need to blow hot or dry air into the chamber 11 itself, thus limiting the energy needed to remove the vapour.

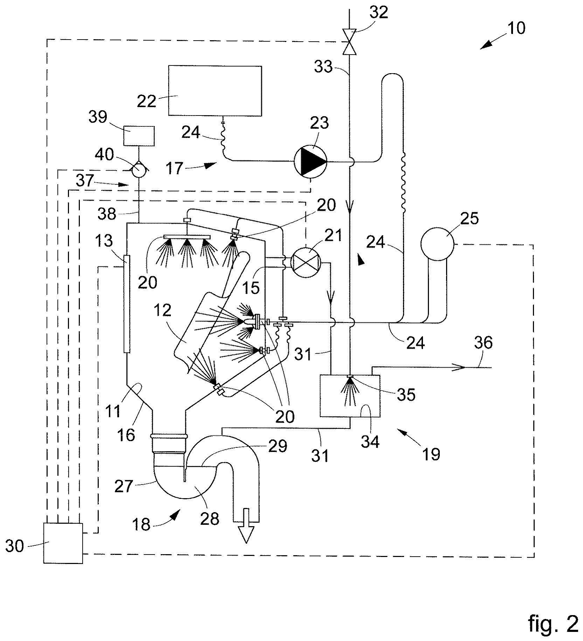

As illustrated in fig. 2, the third circuit 19 may comprise a condensation chamber 34 interposed between the suction fan 21 and the second circuit 18 and fluidically communicating with them thanks to discharge ducts 31. Condensation means may be present in the condensation chamber 34 which facilitate the condensation of the extracted vapour, e.g., water vapour, so that the collected fluid may be discharged into the second circuit 18 in liquid form, e.g., liquid water.

For example, the condensation chamber 34 may comprise at least one second injection means 35, such as a nozzle for injecting a liquid, for example liquid water, into the condensation chamber 34. The at least one second injection means 35 can be fed in a known manner, for example by means of a solenoid valve 32 connected thereto with a second feed duct 33.

In embodiments, the third circuit 19 may also comprise at least one outlet duct 36 configured to constitute an outlet from the condensation chamber 34 alternative to the discharge duct 31. For example, the outlet duct 36 may allow an at least partial recovery of the liquid collected in the condensation chamber 34, i.e. the liquid coming from the second injection means 35 and/or the condensed sanitizing vapour, which may for example be recirculated in the machine 10 or employed elsewhere.

The machine 10 also comprises a ventilation circuit 37, configured to compensate for the extraction of sanitizing fluids from the treatment chamber 11 with clean air.

The ventilation circuit 37 may comprise a filter 39, for example a high efficiency fluid filter such as a HEPA (*High Efficiency Particulate Air filter*) filter, adapted to ensure the entry of clean air into the treatment chamber 11. The filter 39 can be connected to the treatment chamber 11 by means of a suitable ventilation duct 38. The ventilation circuit 37 also comprises a control valve 40 configured to control the incoming air flow.

The suction of external air inside the treatment chamber 11 can take place passively, i.e. as a consequence of the pressure drop in the treatment chamber 11 due to the suction of the sanitizing vapour by the suction fan 21. The use of the ventilation circuit 37 does not entail further energy expenditure.

In embodiments, the control valve 40 can be electrically activated, so that the suction of external air inside the treatment chamber 11 takes place actively. For example, the control unit 30 may be configured to control the selective activation of the control valve 40.

In alternative embodiments, at the same time or after the start of the suction of the sanitizing vapour through the suction fan 21, the door 13 can be opened, partially or totally, so as to allow the entry of fresh external air into the treatment chamber 11, further facilitating the drying of the objects 12.

For example, the door 13 can be opened manually or automatically, in particular according to a desired programming coordinated by the control unit 30 and correlated to a preset time from the start of activation of the suction fan 21.

The first circuit 17, by way of example, comprises a feed pump 23, a plurality of first feed ducts 24 and a vapour generator 25. The feed pump 23 is adapted to supply a liquid, for example water, to the first feed ducts 24. This feed pump 23 can receive the liquid from a feed tank 22 arranged upstream, or from another source, for example a water mains. In the first circuit 17 there may also be injection means for injecting one or more cleaning and/or inerting additives.

The first feed ducts 24 connect the feed pump 23 to the first injection means 20, to supply the washing liquid into the treatment chamber 11. The feed pump 23, through the first feed duct 24 and appropriate inlet and outlet branches (only schematized in the accompanying Figs. 1-2), can also supply liquid to the vapour generator 25 which generates vapour to be injected, for example by means of the same first feed duct 24, into the treatment chamber 11, depending on the steps of the treatment cycle.

While an object 12 is being washed, the washing liquid injected by the first injection means 20, after having involved the surface of said objects 12 to be washed, is evacuated in a known manner through the second washing liquid discharge circuit 18. Subsequently, the step of sanitizing the treatment chamber 11 and said objects 12 is carried out in a known manner with a sanitizing vapour, generated by the vapour generator 25 and injected into the treatment chamber 11, for example through the first injection means 20.

In alternative embodiments, the sanitizing vapour may be supplied by a different feed circuit separated from the first circuit 17 and/or may be injected into the treatment chamber 11 through injection means other than the first injection means 20.

Subsequently, the aforementioned vapour is removed from the treatment chamber 11 by activating the suction fan 21 and, therefore, the objects 12 are dried as described above.

Embodiments described herein also refer to a method for carrying out a treatment cycle, understood as washing and sanitizing, of dirty or contaminated devices, instruments or tools, components or objects 12, such as for example medical, hospital, laboratory devices, instruments or tools or similar or assimilable provenance.

In embodiments, the method includes a step for sanitizing said objects 12 and the same treatment chamber 11 in which they are contained by means of sanitizing vapours which are subsequently extracted by activating a suction fan 21 thus promoting the drying of said objects 12 and of said chamber 11.

Said method can also include a ventilation step, in which the extraction of sanitizing vapours of said chamber 11 is compensated with the suction of external air, possibly previously filtered, into the same chamber 11.

Said method may also include a step of opening the inlet door 13 of said chamber 11, in a coordinated manner with the operation of said suction fan 21, in particular at the same time or after the activation of the suction fan 21, to favour the entry of fresh air from the outside and further promote the drying of said objects 12 and said chamber 11.

Embodiments of this method comprise in particular:
- at least one washing step, in which at least one object 12 to be washed is inserted into a treatment chamber 11 delimited by a containment body 16 and treated with at least one washing liquid;
- at least one step of discharging said washing liquid from the treatment chamber 11 through a second discharge circuit 18;
- at least one sanitizing step with the injection into said chamber 11 of at least one vapour that sanitizes said objects 12 and said chamber 11;
- at least one step of extracting the vapour by suction, for example by means of a suction fan 21, for drying said objects 1 and said chamber 11;
- a ventilation step at the same time and/or after the drying step, in which external air, possibly filtered by a filter 39, enters the treatment chamber 11 through a ventilation circuit 37;
- optionally, a ventilation step at the same time and/or after the drying step, in which the access door 13 to the treatment chamber 11 is opened, allowing the entry of external air that further promotes drying;
- a step of discharging the sanitizing vapour extracted during the drying step by means of a third suction circuit 19 so as to reach the second discharge circuit 18.

In embodiments, any sanitizing vapours extracted from the treatment chamber 11 in the drying step are condensed in a condensation chamber 34 before being discharged, in liquid form, through the second circuit 18.

It is clear that modifications and/or additions of parts may be made to the machine 10 as described heretofore and to the corresponding method, without departing from the field and scope of the present invention, as defined by the claims.

Although the present invention has been described with reference to some specific examples, a person skilled in the art shall certainly be able to achieve other equivalent forms of washing and sanitizing machine, having the characteristics as set forth in the claims and hence all coming within the field of protection defined thereby. In the following claims, the sole purpose of the references in brackets is to facilitate their reading and they must not be considered as restrictive factors with regard to the field of protection defined by the claims.

## Claims

1. Washing and sanitizing machine (10) comprising a containment body (16) which internally delimits a treatment chamber (11) for treating objects (12), as well as a first circuit (17) for feeding washing and/or sanitizing fluids and a second circuit (18) for discharging said washing and/or sanitizing fluids, **characterized in that** it comprises a fan (21), associated with said containment body (16), configured to extract by suction sanitizing vapours of said washing and/or sanitizing fluids from said treatment chamber (11), said machine (10) further comprising a ventilation circuit (37), configured to compensate for the extraction of said sanitizing vapours from said treatment chamber (11) with clean air and provided with a control valve (40) configured to control the incoming air flow.

2. Machine (10) as in claim 1, **characterized in that** said fan (21) is connected downstream with a third suction circuit (19) comprising a discharge duct (31), which places it in fluidic connection with said second circuit (18).

3. Machine (10) as in claim 2, **characterized in that** said discharge duct (31) connects said fan (21) downstream of a siphon (27) of said second circuit (18).

4. Machine (10) as in claim 2, **characterized in that** said third circuit (19) comprises a condensation chamber (34) interposed between said fan (21) and said second circuit (18) and fluidically communicating with them thanks to discharge ducts (31).

5. Machine (10) as in claim 4, **characterized in that** said third circuit (19) comprises an outlet duct (36) from said condensation chamber (34) alternative to said discharge duct (31), configured for the at least partial recovery of the liquid collected in said condensation chamber (34).

6. Machine (10) as in claim 1, **characterized in that** said ventilation circuit (37) comprises a filter (39), adapted to ensure the entry of clean air inside said treatment chamber (11).

7. Machine (10) as in any claim hereinbefore, **characterized in that** it includes a control unit (30) adapted to control the operation thereof.

8. Machine (10) as in claim 7, **characterized in that** said control unit (30) is configured to control the selective activation of said fan (21) as a function of specific steps of a treatment cycle to which the objects (12) are subjected.

9. Machine (10) as in claim 8, **characterized in that** said control unit (30) is further configured to coordinate the opening of a door (13) associated with said treatment chamber (11) according to a desired programming correlated to a pre-set time from the start of the activation of said fan (21).

10. Machine (10) as in claim 8 or 9, **characterized in that** said control unit (30) is configured to control the selective electrical activation of said control valve (40).

11. Method of washing and sanitizing objects (12) in a machine (10) comprising a containment body (16) which internally delimits a treatment chamber (11) for treating said objects (12), said method comprising washing with at least one washing liquid, discharging said at least one washing liquid and sanitizing with at least one sanitizing vapour, **characterized in that** it comprises drying said objects by extracting the at least one sanitizing vapour from said treatment chamber (11) by suction by means of a suction fan (21) and a ventilation step at the same time or after the drying step, in which clean air enters the treatment chamber (11) through a ventilation circuit (37), configured to compensate for the extraction of said sanitizing vapours from said treatment chamber (11) with said clean air and provided with a control valve (40) configured to control the incoming air flow.

12. Method as in claim 11, **characterized in that** it further comprises condensing condensation vapours extracted from said treatment chamber (11) in a condensation chamber (34).

13. Method as in claim 11 or 12, **characterized in that** said ventilation step comprises the introduction of filtered external air from a filter (39).

14. Method as in claim 11 or 12, **characterized in that** it comprises the opening of a door (13) associated with said treatment chamber (11) at the same time or after the activation of said suction fan (21), for the entry of fresh air from the outside and promoting the drying of the objects.
